# FASCICULE DE BREVET EUROPEEN

(11) **EP 1 668 062 B1**
(45) Date de publication et mention de la délivrance du brevet: **25.03.2009**
(21) Numéro de dépôt: 04817101.1
(22) Date de dépôt: 28.09.2004
(51) Int. Cl.: C08G 73/02, C08G 69/10, A61K 9/20, C08L 79/02, C08L 77/04

(54) **HOMOPOLYAMINOACIDES TELECHELIQUES FONCTIONNALISES PAR DES GROUPEMENTS HYDROPHOBES ET LEURS APPLICATIONS NOTAMMENT THERAPEUTIQUES**
MIT HYDROPHOBEN GRUPPEN TELECHELISCHE FUNKTIONALISIERTE HOMOPOLYAMINOSÄURE UND DEREN ANWENDUNGEN, INSBESONDERE IM THERAPEUTISCHEN BEREICH
TELECHELIC HOMOPOLYAMINO ACIDS FUNCTIONALIZED BY HYDROPHOBIC GROUPS AND USES, PARTICULARLY THERAPEUTIC, THEREOF

(30) Priorité: 03.10.2003 FR 0350641
(43) Date de publication de la demande: 14.06.2006
(73) Titulaire: FLAMEL TECHNOLOGIES, 69200 Vénissieux (FR)
(72) Inventeur: SOULA, Rémi, F-69007 Lyon (FR); CHAN, You-Ping, F-69008 Lyon (FR); SOULA, Gérard, F-69330 Meyzieu (FR); BREYNE, Olivier, F-69004 Lyon (FR)
(74) Mandataire: Fleurance, Raphaël
(86) Numéro de dépôt international: PCT/FR2004/050465
(87) Numéro de publication internationale: WO 2005/033181

(56) Documents cités:
- WO-A-00/30618
- WO-A-00/78791
- WO-A-02/098951
- US-A- 4 888 398

## Description

La présente invention concerne des nouveaux matériaux à base d'homopolyaminoacides biodégradables, utiles notamment pour la vectorisation de principe(s) actif(s) (PA).

L'invention vise aussi de nouvelles compositions pharmaceutiques, cosmétiques, diététiques ou phytosanitaires à base de ces homopolyaminoacides. Ces compositions peuvent être du type de celles permettant la vectorisation de PA et se présentant, de préférence, sous forme d'émulsions, de micelles, de particules, de gels, d'implants ou de films.

Les PA considérés sont, avantageusement, des composés biologiquement actifs et qui peuvent être administrés à un organisme animal ou humain par voie orale, parentérale, nasale, vaginale, oculaire, sous-cutanée, intraveineuse, infra-musculaire, intradermique, intrapéritonéale, intracérébrale, buccale, etc.
Les PA plus particulièrement, mais non limitativement, concernés par l'invention sont des protéines, des glycoprotéines, des peptides, des polysaccharides, des lipopolysaccharides, des oligo ou des polynucléotides, et des molécules organiques. Mais il peut aussi s'agir de produits cosmétiques ou de produits phytosanitaires, tels que des herbicides, des insecticides, des fongicides, etc.

Dans le domaine de la vectorisation des principes actifs notamment médicamenteux, il existe un besoin, dans beaucoup de cas :
- de les protéger contre la dégradation (hydrolyse, précipitation sur site, digestion enzymatique etc..) jusqu'à ce qu'ils atteignent leur site d'action,
- et/ou de contrôleur leur vitesse de libération afin de maintenir un niveau thérapeutique sur une durée définie,
- et/ou de les véhiculer (en les protégeant) au site d'action.

A ces fins, plusieurs types de polymères ont été étudiés et certains sont même disponibles commercialement. On peut citer par exemple les polymères du type polylactique, polylactique-glycolique, polyoxyethylène-oxypropylène, polyaminoacide ou encore polysaccharide. Ces polymères constituent des matières premières permettant de fabriquer, par exemple, des implants massiques, des microparticules, des nanoparticules, des vésicules, des micelles ou des gels. Outre le fait que ces polymères doivent être adaptés à la fabrication de tels systèmes, ils doivent également être biocompatibles, non-toxiques, non-immunogènes, économiques et ils doivent pouvoir être facilement éliminés du corps et/ou être biodégradables. Sur ce dernier aspect, il est de surcroît essentiel que la biodégradation dans l'organisme génère des produits non-toxiques.

A titre d'illustration de l'art antérieur concernant des polymères employés comme matières premières pour la réalisation de systèmes de vectorisation de PA, divers brevets ou demandes de brevet ou articles scientifiques sont évoqués ci-après.

Le brevet US-B-4,652,441 décrit des microcapsules de polylactide encapsulant l'hormone LH-RH. Ces microcapsules sont produites en préparant une émulsion eau-dans-huile-dans-eau et comprennent une couche interne aqueuse contenant l'hormone, une substance (gélatine) fixant cette dernière, une couche huileuse de polylactide, ainsi qu'une couche externe aqueuse (alcool polyvinylique). La libération du PA peut se faire sur une période de plus de deux semaines après injection sous-cutanée.

Le brevet US-B-6,153,193 décrit des compositions à base de micelles de poly(oxyéthylène)-poly(oxypropylène) amphiphiles, pour la vectorisation d'anti-cancéreux tel que l'adriamycine.

Akiyoshi et al. (J. Controlled Release 1998, 54, 313-320) décrivent des pullulants qui sont rendus hydrophobes par greffage de cholestérol et qui forment des nanoparticules dans l'eau. Ces nanoparticules aptes à se complexer de manière réversible avec l'insuline, forment des suspensions colloïdales stables.

Le brevet US-B-4,351,337 décrit des copolyaminoacides amphiphiles, à base de leucine et de glutamate, utilisables sous forme d'implants ou de microparticules pour la libération contrôlée de principes actifs. La libération de ces derniers peut se faire sur une durée très longue dépendant de la vitesse de dégradation du polymère.

Le brevet US-B-4,888,398 décrit des polymères à base de polyglutamate ou polyaspartate, et éventuellement polyleucine, avec des groupements pendants de type alkyloxycarbonylméthyle, placés de façon aléatoire sur la chaîne polysminoacide. Ces polyaminoacides, greffés par des groupements latéraux e.g. méthoxycarbonylméthyle, sont utilisables sous forme d'implants biodégradables contenant un PA à libération prolongée.

Le brevet US-B-5,904,936 décrit des nanoparticules obtenues à partir d'un polymère bloc polyleucine-polyglutamate, aptes à former des suspensions colloïdales stables et capables de s'associer spontanément avec des protéines biologiquement actives sans les dénaturer. Ces dernières peuvent ensuite être lisérées in vivo de manière contrôlée, sur une longue période.

Le brevet US-B-5,449,513 décrit des copolymères blocs amphiphiles comprenant un bloc polyoxyéthylène et un bloc polyaminoacide, par exemple poly(bêta-benzyl-L-aspartate). Ces polymères polyoxyéthylène-polybenzylaspartate forment des micelles qui sont aptes à encapsuler des molécules actives hydrophobes telles que l'adriamycine ou l'indométhacine.

La demande de brevet WO-A-99/61512 décrit des polylysines et des polyornithines fonctionnalisées par un groupe hydrophobe (acide palmitique relié à la polylysine ou ornithine) et un groupe hydrophile (polyoxyéthylène). Ces polymères, par exemple la polylysine greffée avec des chaînes polyoxyéthylène et palmitoyle formes en présence de cholestérol, des vésicules capables d'encapsuler la doxorubicine ou l'ADN. Ces polymères à base de polylysines sont cationiques en milieu physiologique.

La demande de brevet WO-A-00/30618, de la demanderesse, décrit des polymères blocs ou aléatoires poly(glutamate de sodium)-poly(glutamate de méthyle, d'éthyle, d'hexadécyle ou de dodécyle), aptes à former des suspensions colloïdales stables et capables de s'associer spontanément avec des protéines biologiquement actives sans les dénaturer. Ces dernières peuvent ensuite être libérée in vivo de manière contrôlée, sur une longue période. Ces capolyaminoacides amphiphiles sont modifiés par la présence d'une chaîne latérale alkyle hydrophobe. Ces groupements alkyles sont greffés de façon covalente sur le polymère *via* une fonction ester. Ces polymères sont anioniques en milieu physiologique.

Au sein de cet art antérieur relatif aux systèmes de vectorisation, il existe un certain nombre de références concernant des polymères hydrophiles comportant des groupements hydrophobes en bout de chaînes.
- Le brevet FR 2 533 209, décrit des lipopeptides constitué d'une chaîne hydrophobe comprenant 8 à 24 atomes de carbones et d'une chaîne peptidique hydrophile ou rendue hydrophile. Ces produits sont notamment utiles comme émulsifiants ou comme cristaux liquides.

Dans le même esprit et dans le domaine technique des liposomes, on peut citer :
- La demande de brevet WO-A-02/098951 qui décrit des polyaminoacides ou certains de leurs dérivés ayant un groupement lipidique sur l'un des deux bouts de chaîne. Il peut s'agir par exemple de poly(gamma-L-benzyl-L-glutamate) ou de poly(N(2-hydroxyéthyl)-L-glutamine) porteur d'un groupement terminal heptadécyloctadécylamine. Ces polymères sont utiles à la préparation des liposomes.
- Le brevet US-B-5,534,241 divulgue des composés amphiphiles constitués par des restes polylysine substitués, dans la chaîne, par des radicaux chélatants du type acide diéthylènetriaminepentaacétique et, à l'une des extrémités de la chaîne, par un groupement lipophile formé par de la N-glutarylphosphatidyléthanolamine (NGPE). Ces composés sont destinés à être incorporés dans la membrane bicouche de liposomes.
- Il en va de même pour les polymères amphiphiles décrits dans le brevet US-B1-6,284, 267. Ces polymères amphiphiles sont des polymères hydrophiles, linéaires, branchés ou en étoile, avec au moins deux groupements hydrophobes liés à leurs extrémités. Ce brevet concerne essentiellement des polymères hydrophiles neutres à base de polyéthylène glycol, comme en témoigne tous les exemples de ce brevet. Or, ce type de polymère n'est pas biodégradable ce qui constitue un inconvénient majeur.
   Par conséquent, ce brevet ne décrit pas et suggère encore moins l'utilisation d'un polyaminoacide, linéaire et anionique tel qu'un polyglutamate ou un polyaspartate, à titre de partie polymère hydrophile dans un polymère amphiphile téléchélique.

Ainsi, même si de très nombreuses solutions techniques sont développées et proposées dans l'art antérieur pour la vectorisation des principes actifs médicamenteux, la réponse à l'ensemble des exigences est difficile à obtenir et demeure non satisfaisante. Plus spécifiquement, on a pu identifier un besoin non satisfait en un matériau biodégradable pour la réalisation de particules de vectorisation de principes actifs, ce matériau devant être capable de former une suspension aqueuse de nano- ou microparticules de vectorisation propres à s'associer réversiblement à des principes actifs.

Dans ce contexte, l'un des objectifs essentiels de la présente invention est de fournir de nouveaux polyaminoacides, amphiphiles, linéaires et amoniques à pH physiologique animal (par exemple de l'ordre de 7,4), qui représentent un perfectionnement par rapport à ceux décrits dans le brevet FR-A-2 533 209 ou le brevet US-B1-6,284,267, notamment en termes de taux d'association d'une protéine et de biodégradabilité.

Un autre objectif essentiel de la présente invention est que ces polymères soient aptes à être utilisés pour la vectorisation de PA et permettent de satisfaire de manière optimale à toutes les spécifications du cahier des charges, à savoir notamment :
o capacité :
   ■ à former aisément et économiquement des suspensions colloïdales aqueuses stables,
   ■ à s'associer facilement avec de nombreux principes actifs,
   ■ et à libérer ces principes actifs in vivo,
o biocompatibilité,
o biodégradabilité,
o stabilité à l'hydrolyse.
Cet objectif, parmi d'autres, est atteint par la présente invention qui concerne tout d'abord un homopolyaminoacide amphiphile, linéaire et anionique caractérisé en ce que ses deux extrémités sont porteuses de groupements hydrophobes, identiques ou différents entre eux et en ce qu'il peut être symbolisé par la formule générale schématique suivante:

GH-X-PAA-Y-GH

avec:
- GH étant un Groupement Hydrophobe,
- X, Y étant indépendamment un lien correspondant à une liaison covalente ou à un radical polyvalent issu d'une entité chimique distincte du précurseur de GH,
- PAA étant une chaîne homopolyaminoacide hydrophile et anionique.

Il est du mérite de la demanderesse d'avoir eu l'idée de combiner, de façon tout à fait judicieuse et avantageuse, des homopolyaminoacides particulier linéaires, biodégradables et anioniques (e.g. polyAsp ou polyGlu) avec des groupements hydrophobes placés sur les bouts de chaîne du PAA.

Ces nouveaux homopolymères amphiphiles se sont avérés être particulièrement bien adaptés pour la vectorisation des protéines.
Au sens de l'invention:
➢ le terme "*homopolyaminoacide* " couvre, d'une part les PAA comportant un seul type d'unité "acide aminé" (par exemple soit des unités glutamiques ou glutamates, soit des unités aspartiques ou aspartates) et, d'autre part, aussi bien les oligoaminoacides comprenant de 2 à 20 unités "acide aminé" que les homopolyaminoacides comprenant plus de 20 unités "acide aminé";
➢ le terme "*unité acide aminé*" vise un motif monomérique ou non formé par un squelette d'un acide aminé donné, quelles que puissent être les substitutions pour sautant qu'elles ne modifient pas la nature de l'acide aminé concerné.

Ces homopolymères présentent des propriétés surprenante d'association et/ou d'encapsulation avec un ou plusieurs principes actifs, en comparaison avec des produits analogues.
De plus, ils sont facilement dégradés, en présence d'enzymes, en catabolites/métabolites non toxiques (acides aminés).

Au sens de l'invention et dans tout le présent exposé, les termes "association" ou "associer" employés pour qualifier les relations entre un ou plusieurs principes actifs et les homopolyaminoacides, signifient en particulier que le ou les principes actifs sont liés au(x) homopolyaminoacide(s) notamment par une liaison faible, par exemple par liaison ionique et/ou par contact hydrophobe, et/ou sont encapsulés par le ou les homopolyaminoacides.

De préférence, les homopolyaminoacides selon la présente invention sont des homooligomères ou des homopolymères comprenant des unités d'alpha-L-glutamate et/ou d'alpha-L-glutamique ou des unités d'alpha-L-aspartate et/ou d'alpha-L-aspartique.

S'agissant les groupements hydrophobes GH, ils sont avantageusement et judicieusement sélectionnés dans le groupe comprenant :
■ les alkyles linéaires ou ramifiés en C8 à C30 pouvant comporter éventuellement au moins une insaturation et/ou au moins un hétéroatome,
■ les alkylaryles ou arylalkyles en C8 à C30 pouvant comporter éventuellement au moins une insaturation et/ou au moins un hétéroatome,
■ et les (poly)cycliques en C8 à C30 pouvant comporter éventuellement au moins une insaturation et/ou au moins un hétéroatome.

Dans le cas où les liens X,Y sont des liaisons covalentes directes, les précurseurs des GH sont, en pratique et sans que cela ne soit limitatif, choisis dans le groupe comprenant les alcools, les acides carboxyliques et les amines, ces composés pouvant être fonctionnalisés facilement par l'homme de l'art.
Les GH sont alors liés aux extrémités PAA par des liaisons amide, ester, carbonate, carbamate ou urée.

Dans le cas où les liens X,Y sont des radicaux polyvalents (par exemple divalents) issus d'une entité chimique distincte du précurseur de GH, les précurseurs des GH sont de préférence sélectionnés parmi les mêmes espèces que dans le cas où X,Y = liaison covalente directe.
Mais cette fois les GH forment une liaison covalente avec le lien (ou radical d'espacement) X,Y et ne sont pas directement liés aux extrémités PAA.
X,Y est ainsi un pont qui relie, d'une part, GH et, d'une autre part, une extrémité de PAA.
X,Y sont choisis, indépendamment, parmi les radicaux issus de composés fonctionnels propres à réagir avec le ou les groupements fonctionnels de GH et les groupements N-terminaux et C-terminaux de la partie PAA. Ces composés fonctionnels peuvent être avantageusement ceux appartenant an groupe comportant: les "acides aminés. différents de l'unité monomérique constitutive de la partie homopolymère PAA, les aminoalcools, les diamine, diacides, diols et hydroxyacides.
En tout état de cause, les liaisons chimiques PAA-(X ou Y) et (X ou Y)-GH sont elles aussi préférablement des liaisons amide, ester, carbonate, carbamate ou urée.

Selon un mode préféré de réalisation de l'invention, l'homopolyaminoacide répond à l'une des formules générales (I), (II) et (III) suivantes : 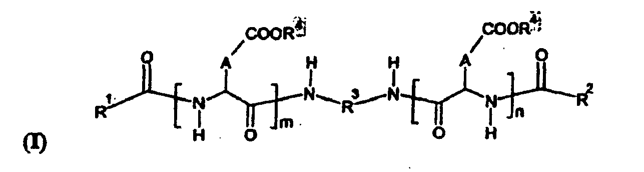 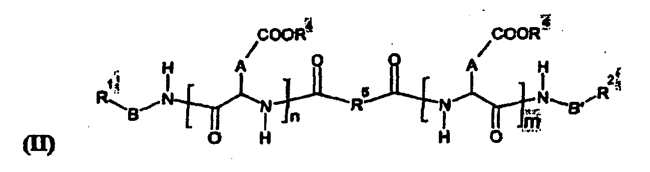 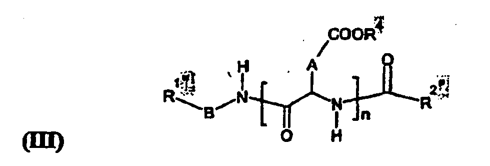 dans lesquelles :
■ R¹CO-, R²CO-, R^{1'}, R^{2'}, R^{1"} et R^{2"} représentent indépendamment un groupement hydrophobe en C8 à C30
■ R³ est un groupement alkyle linéaire en C2 à C6
■ R⁴ est un H ou une entité cationique, de préférence sélectionnée dans le groupe comprenant :
   - les cations métalliques avantageusement choisis dans le sous-groupe comprenant : le sodium, le potassium, le calcium, le magnésium ;
   - les cations organiques avantageusement choisis dans le sous-groupe comprenant :
      - les cations à base d'amine,
      - les cations à base d'oligoamine,
      - les cations à base de polyamine (la polyéthylèneimine étant particulièrement préférée),
      - les cations à base d'acide(s) aminé(s) avantageusement choisis dans la classe comprenant les cations à base de lysine ou d'arginine,
   - ou les polyaminoacides cationiques avantageusement choisis dans le sous-groupe comprenant la polylysine ou l'oligolysine,
■ R⁵ est un groupement alkyle, dialcoxy ou diamine en C2 à C6
■ A représente indépendament un -CH₂- (unité aspartique) ou -CH₂-CH₂-(unité glutamique)
■ B est un lien formé par une liaison covalente directe ou un reste acide aminé répondant à la formule suivante : 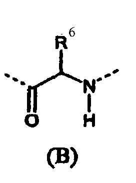
dans laquelle R⁶ est un radical caractéristique d'un acide aminé naturel, de préférence choisi dans le groupe comprenant : H (B est alors un reste glycine), Méthyle (B est alors un reste alanine), isobutyle (B est alors un reste leucine), isopropyle (B est alors un reste valine) ou CH₂Ph (B est alors un reste phénylalanine)
■ n+m est défini comme le degré de polymérisation et varie de 3 à 1000, de préférence entre 20 et 300.
Plus Préférentiellement, les groupements hydrophobes R¹CO-, R²CO-, R^{1'}, R^{2'}, R^{1"} et R^{2"} sont des groupements :
■ alkyles linéaires ou ramifiés en C8 à C30 pouvant comporter éventuellement au moins une insaturation et/ou au moins un hétéroatome,
■ alkylaryles ou arylalkyles en C8 à C30 pouvant comporter éventuellement au moins une insaturation et/ou au moins un hétéroatome, ou
■ (poly)cycliques en C8 à C30 pouvant comporter éventuellement au moins une insaturation et/ou au moins un hétéroatome.
En pratique, le groupement hydrophobe GH est par exemple un groupement choisi dans le groupe comprenant les espèces suivantes: palmitate, stéarate, cholestéryle, tocophéryle.

Selon un autre mode de définition, les homopolyaminoacides selon l'invention ont une masse molaire qui se situe entre 2 000 et 100 000 g/mole, et de préférence entre 5 000 et 40 000 g/mole.

De manière remarquable, les homopolyaminoacides de l'invention sont susceptibles d'être utilisés de plusieurs façons selon la nature des groupements hydrophobes et le degré de polymérisation de l'homopolyaminoacide. Les méthodes de mise en forme d'un polymère pour l'encapsulation d'un principe actif sous les diverses formes visées par l'invention sont connues de l'homme de l'art. Pour plus de détails, on peut se référer, par exemple à ces quelques références particulièrement pertinentes :
"Microspheres, Microcapsules and Liposomes; vol 1. Preparation and chemical applications" Ed. R. Arshady, Citus Books 1999. ISBN : 0-9532187-1-6.
*"*Sustained-Release Injectable Products" Ed. J. Senior et M. Radomsky, Intorpharm Press 2000. ISBN : 1-57491-101-5.
"Colloidal Drug Delivery Systems" Ed. J. Kreuter, Marcel Dekker, Inc. 1994. ISBN : 0-8247-9214-9.
"Handbook of Pharmaceutical Controlled Release Technology" Ed. D.L. Wise, Marcel Dekker, Inc. 2000. ISBN : 0-8247-0369-3.

Ces homopolyaminoacides sont en outre extrêmement intéressants du fait que, selon la longueur de l'homopolymère (degré de polymérisation) et la nature des groupements hydrophobes, ils se dispersent dans l'eau à pH 7,4 (par exemple avec un tampon phosphate) pour donner des solutions ou des suspensions colloïdales ou des gels structurés ou non, en fonction de la concentration en homopolymères. De plus, les polyaminoacides (sous forme de particules ou non), peuvent encapsuler ou s'associer aisément avec des principes actifs tels que des protéines, peptides ou petites molécules. La mise en forme préférée est celle décrite dans la demande de brevet WO-A-00/30618 de la demanderesse et qui consiste à disperser l'homopolymère dans l'eau et à incuber la solution en présence d'un principe actif (PA). Cette solution colloïdale de particules de vectorisation constituées des homopolyaminoacides selon l'invention, peut ensuite être filtrée sous 0,2 µm puis directement injectée à un patient.

Quand le rapport hydrophile/hydrophobe diminue, l'homopolylmère peut alors former des microparticules capables d'associer ou d'encapsuler des PA. Dans ce contexte, la mise en forme des microparticules peut se faire en co-solubilisant le PA et l'homopolymère dans un solvant organique approprié puis le mélange est précipité dans l'eau. Les particules sont ensuite récupérées par filtration et peuvent ensuite être utilisées pour une administration par voie orale (sous forme de gélule, sous forme compactée et/ou enrobée ou bien encore sous forme dispersée dans une huile) ou par voie parentérale après redispersion dans l'eau.

Selon une variante, l'homopolymère peut être solubilisé dans un solvant biocompatible tel que la N-méthylpyrrolidane ou une huile appropriée telle que le Mygliol® puis injecté en intramusculaire ou sous-cutané ou dans une tumeur. La diffusion du solvant ou de l'huile conduit à la précipitation de l'homopolymère sur le site d'injection et forme ainsi un dépôt. Ces dépôts assurent ensuite une libération contrôlée par diffusion et/ou par érosion et/ou par dégradation hydrolytique ou enzymatique de l'homopolymère.

Indépendamment du fait que la forme microparticulaire de l'homopolyaminoacide selon l'invention est préférée, les homopolymères de l'invention, sous forme neutre ou ionisée, sont de façon plus générale, utilisables seuls ou dans une composition liquide, solide ou gel et dans un milieu aqueux ou organique.

Il convient de comprendre que l'homopolymére à base de polyaminoacides contient des fonctions carboxyliques qui sont soit neutres (forme COOH), soit ionisées (anion COO⁻), selon le pH et la composition. Pour cette raison, la solubilité dans une phase aqueuse est directement fonction du taux de COOH libre de l'homopolymère (non greffé par le motif hydrophobe) et du pH. En solution aqueuse, le contre-cation peut être un cation métallique tel que le sodium, le calcium ou le magnésium, ou un cation organique tel que la triéthanolamine, la tris(hydroxyméthyl)-aminométhane ou une polyamine tel que la polyéthylèneimine.

Les homopolymères de l'invention sont par exemple obtenus par des méthodes connues de l'homme de l'art Tout d'abord, rappelons que pour l'obtention de polyaminoacide de type alpha, la technique la plus courante est basée sur la polymérisation d'anhydrides de N-carboxy-aminoacides (NCA), décrites, par exemple, dans l'article "Biopolymers, 1976, 15, 1869 et dans l'ouvrage de H.R. Kricheldorf "alpha-Aminoacid-N-carboxy Anhydride and related Heterocycles" Springer Verlag (1987). Le dérivé de NCA est de préférence NCA-Glu-O-Bz (Bz = Benzyle), car le groupement benzyle peut être sélectivement hydrolysé sans toucher d'autres fonctions chimiques des homopolymères ou du groupement hydrophobe.

A. titre d'exemple, un homopolymère appartenant à la structure générale (I) peut être obtenu notamment selon le schéma suivant (avec R₁ = R₂ = groupement stéarate, R₃ = -(CH₃)₂- et le polymaminoacide est un polyglutamate).

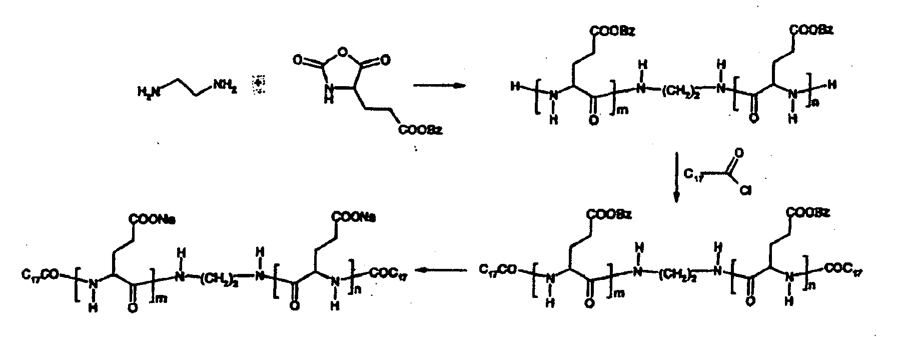

Un homopolymère de structure (II) peut être obtenu notamment par la séquence de réaction suivante : initiation d'une polymérisation de NCA-Glu-O-Bz par un groupement hydrophobe ayant une fonction amine pour donner un intermédiaire (II-A), terminé par une fonction amine, puis, couplage du produit intermédiaire (II-A) avec un composé difonctionnel approprié tel qu'un dichlorure d'acide, un diisocyanate ou un dichloroformiate.

Un homopolymère de structure (III) peut être quant à lui obtenu notamment par réaction de l'intermédiaire (II-A) avec un groupement hydrophobe fonctionnalisé tel qu'un chlorure d'acide ou un chloroformiate.

A titre d'exemple un composé intermédiaire de type (II A) peut être obtenu notamment selon le schéma suivant (R^{1'} est le dodécanol et B est la leucine). 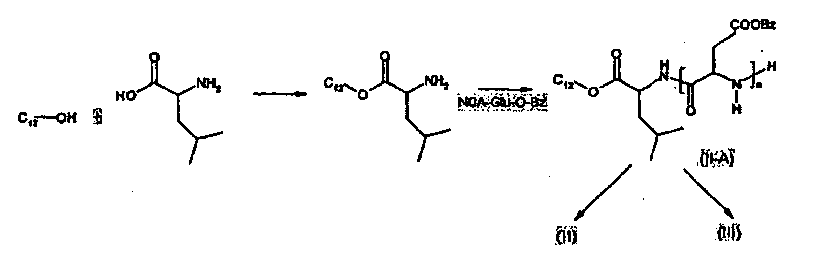 Il doit être observé que le degré de polymérisation est défini par le rapport molaire de l'initiateur sur celle du monomère.

Selon un autre de ses aspects, l'invention vise une composition pharmaceutique, cosmétique, diététique ou phytosanitaire comprenant au moins un homopolyaminoacide tel que défini ci-dessus et éventuellement au moins un principe actif, qui peut être thérapeutique, cosmétique, diététique ou phytosanitaire.

Suivant une disposition intéressante de l'invention, le principe actif est associé au(x) homopolyaminoacide(s) par une ou plusieurs liaisons autre(s) qu'une (ou que des) liaison(s) chimique(s) covalente(s).

Les techniques d'association d'un ou de plusieurs PA aux homopolyaminoacides greffés selon l'invention, sont décrites notamment dans la demande de brevet WO-A-00/30618. Elles consistent à incorporer au moins un principe actif dans le milieu liquide contenant des particules PV, de manière à obtenir une suspension colloïdale de PV chargées en ou associées avec un ou plusieurs principe(s) actif(s) PA. Cette incorporation, qui conduit à un piégeage de PA par les PV, peut être réalisée de la manière suivante :
- mise en solution aqueuse de PA, puis ajout des PV, soit sous forme de suspension colloïdale, soit sous forme de PV isolées (lyophilisat ou précipité) ;
- ou ajout de PA, soit en solution, soit à l'état pur ou préformulé, à une suspension colloïdale de particules PV, éventuellement préparée extemporanément par la dispersion de PV sèches dans un solvant approprié, tel que l'eau.

De préférence, le principe actif est une protéine, une glycoprotéine, une protéine liée à une ou plusieurs chaînes polyalkylèneglycol (de préférence PolyÉthylèneGlycol (PBG) : "protéine-PEGylée"), un polysaccharide, un liposaccharide, un oligonucléotide, un polynucléotide ou un peptide.

Selon une variante, le principe actif est une "petite" molécule organique hydrophobe, hydrophile ou amphiphile.
Au sens du présent exposé, une "petite" molécule est notamment une petite molécule non protéinique.

Comme exemples de PA susceptibles d'être associés aux homopolyaminoacides selon l'invention, qu'ils soient ou non sous forme de (nano ou micro)particules, on peut citer :
o les protéines telles que l'insuline, les interférons, les hormones de croissance, les interleukines, l'érythropoïétine ou les cytokines;
o les peptides telles que la leuprolide ou la cyclosporine ;
o les petites molécules telles que celles appartenant à la famille des anthracyclines, des taxoïdes ou des camptothécines ;
o et leurs mélanges.

Selon un mode de réalisation, la composition de l'invention est sous forme d'un gel, d'une solution, d'une suspension, d'une émulsion, de micelles, de nanoparticules, de microparticules, d'un implant, d'une poudre ou d'un film.

Suivant l'une de ses formes particulièrement préférées, la composition, chargée ou non en principe actif(s), est une suspension colloïdale stable de nanoparticules et/ou de microparticules et/ou de micelles d'homopolyaminoacides, dans une phase aqueuse.

Selon une autre mode de réalisation, la composition de l'invention est sous forme de solution dans un solvant biocompatible et peut être injectée par voie sous-cutanée, intramusculaire ou dans une tumeur.

La composition selon invention, dès lors qu'elle est pharmaceutique, peut être administrée par voie orale, parentérale, nasale, vaginale, oculaire, sous-cutanée, intraveineuse, intramusculaire, intradermique, intrapéritonéale, intracérébrale ou buccale.

Il est également envisageable que la composition soit sous forme de solution dans un solvant biocompatible, susceptible d'être injectée en sous-cutané, intramusculaire ou dans une tumeur.

Selon une autre variante, la composition selon l'invention est formulée de telle sorte qu'elle soit apte à former un dépôt au site d'injection.

L'invention vise aussi des compositions qui comprennent des homopolyaminoacides selon l'invention et des principes actifs et qui sont susceptibles d'être utilisées pour la préparation :
- de médicaments, en particulier pour administration orale, nasale, vaginale, oculaire, sous-cutanée, intraveineuse, intramusculaire, intradermique, intrapéritonéale ou intracérébrale, les principes actifs de ces médicaments pouvant être, notamment, des protéines, des glycoprotéines, des protéines liées à une ou plusieurs chaînes polyalkylèneglycol {par exemple PolyÉthyléneGlycol (PEG), on parle alors de protéines "PEGylées"}, des peptides, des polysaccharides, des liposaccharides, des oligonucléotides, des polynucléotides et des petites molécules organiques hydrophobes, hydrophiles ou amphiphiles,
- et/ou des nutriments,
- et/ou de produits cosmétiques ou phytosanitaires.

Selon encore un autre de ses aspects, l'invention vise un procédé de préparation:
- de médicaments, en particulier pour administration orale, nasale, vaginale, oculaire, sous-cutanée, intraveineuse, intramusculaire, intradermique, intrapérito-néale ou intracérébrale, les principes actifs de ces médicaments pouvant étre, notamment, des protéines, des glycoprotéines, des protéines liées à une ou plusieurs chaînes polyalkylèneglycol {par exemple PolyÉthylèneGlycol (PEG), on parle alors de protéines "PEGylées"}, des peptides, des polysaccharides, des liposaccharides, des oligonucléotides, des polynucléotides et des petites molécules organiques hydrophobes, hydrophiles ou amphiphiles,
- et/ou des nutriments,
- et/ou de produits cosmétiques ou phytosanitaires,
ce procédé étant caractérisé en ce qu'il consiste essentiellement à mettre en oeuvre au moins un homopolyaminoacide tel que défini ci-dessus et/ou la composition elle aussi décrite supra.

L'invention concerne également une méthode de traitement thérapeutique consistant essentiellement à administrer la composition telle que décrite dans le présent exposé, par voie orale, parentérale, nasale, vaginale, oculaire, sous-cutanée, intraveineuse, intramusculaire, intradermique, intrapéritonéale, intracérébrale ou buccale.

Suivant une variante particulière de l'invention, cette méthode de traitement thérapeutique consiste essentiellement à mettre la composition telle que décrite supra sous forme de solution dans un solvant biocompatible puis à l'injecter en sous-cutané, intramusculaire ou dans une tumeur, de préférence de manière à ce qu'elle forme un dépôt au site d'injection.

L'invention sera mieux comprise et ses avantages et variantes de mise en oeuvre ressortiront bien des exemples qui suivent et qui décrivent la synthèse des homopolyaminoacides téléchéliques, leur transformation en système de vectorisation de PA (suspension colloïdale aqueuse stable) et la démonstration de la capacité d'un tel système de s'associer à une protéine pour former des compositions pharmaceutiques.

### Exemple 1 : Synthèse d'un t-pGluONa C18/C18

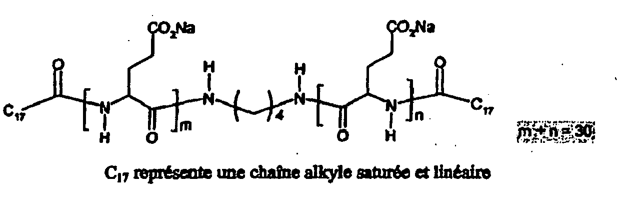

### Etape 1 : Polymérisation

Dans un réacteur de 1 L sous flux d'azote, 39,5 g de NCA GluOBz sont dissous dans 495 ml de NMP à 40°C. 0,33 g de 1,4-butanediamine sont solubilisés dans 5 ml de NMP puis ajoutés au milieu réactionnel. La polymérisation est arrêtée à 90% de conversion du NCA par ajout de HCl (4M dans le dioxane, 5,0 ml). Le polymère est précipité dans un mélange méthanol/éther diisopropylique (700/2800 ml), filtré puis lavé au méthanol (2 xl L) et à l'éther diisopropylique (2 x 1 L). Le produit est enfin séché dans une étuve sous vide à 40°C. 10,8 g du polymère téléchélique t-pGluOBz.2HCl sont obtenus.
La masse molaire en nombre (Mn) (déterminé par GPC) est de 4,5 kg/mol en équivalents PMMA.

### Etape 2 : Greffage sur les amines terminales

6 g du précédent polymère sont ensuite dissous, à température ambiante, dans 300 ml de THF. Cette solution est refroidie à 0°C avant de procéder à l'ajout du chlorure d'acide palmitique (0,89 g). Enfin, 0,47 g de triéthylamine sont à leur tour ajoutés. Le milieu est ensuite remis à température ambiante pour une heure. A la fin de la réaction, le milieu réactionnel est versé dans 2,1 L d'éther diisopropylique. Le précipité est filtré, lavé à l'éthanol à 96% (3x300 ml) puis à l'éther diisopropylique (3x300 ml). Le produit est enfin séché dans une étuve sous vide à 40°C. 6,3 g du polymère t-pGluOBz-C18 sont obtenus. La Mn (déterminé par GPC NMP) est de 6,1 kg/mol en équivalents PMMA.

### Etape 3 : Hydrolyse des esters benzyliques

6 g du précédent polymère sont dissous dans 46 ml de TFA à température ambiante. La solution est ensuite refroidie à 0°C avant de procéder à l'ajout au goutte à goutte du HBr (à 30% en poids dans l'acide acétique, 20,6 ml soit 4 équivalents). A la fin de l'ajout, le milieu est agité 4 heures à température ambiante. La fin de la réaction est vérifiée par RMN ¹H du milieu réactionnel dans le TFA*-d*. Le milieu réactionnel est ensuite versé dans un mélange eau-éther diisopropylique (50-50, volume total de 324 ml). Le polymère est ensuite filtré sur fritté et lavé à l'éther diisopropylique (3x100 ml). Le polymère est séché 48 h à 40°C sous vide. 3,3 g du polymère sont obtenus.
La présence des groupements hydrophobes greffés sur les extrémités du polymère est confirmée par RMN ¹H dans le TFA*-d* et le degré de polymérisation est de 30 (la valeur nominale est de 30). La Mn (déterminé par GPC NMP) est de 6,1 kg/mol en équivalents PMMA.

### Etape 4 : Mise en suspension du polymère dans l'eau

2,7 g du polymère précédent sont mis en suspension dans 100 ml d'eau déminéralisée. 21 ml d'une solution de NaOH 1N sont lentement ajoutés. La neutralisation est terminée lorsque tout le polymère s'est solubilisé et que le pH se situe autour de 7,4. On obtient une solution limpide à l'oeil et stable dans le temps à température ambiante.

### Exemple 2 : Synthèse d'un t-pGluONa PheOC12/C12

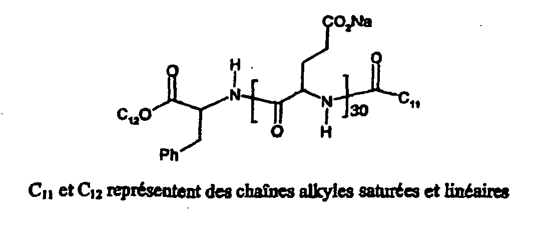

### Etape 1: Polymérisation

Dans un réacteur de 500 ml sous azote, 192 ml de NMP sont chauffés à 40°C. 60g de NCA GluOBz sont ensuite dissous dans ce solvant Lorsque le milieu est homogène, une solution de l'amorceur dans la NMP (2,28 g de PheOCl2 dans 25 ml à 40°C) est introduite dans le milieu réactionnel. La réaction est suivie par IR pour estimer la conversion du NCA. A 90%, la réaction est stoppée par ajout d'un excès d'HCl (4M dans le dioxane, 4,27 ml). Le milieu réactionnel est alors lentement coulé dans 4,5 L d'eau. Le polymère précipité est ensuite filtré, lavé au méthanol acide (2x310 ml) puis à l'éther diisopropylique (2x310 ml). Le produit est enfin séché dans une étuve sous vide à 40°C. 39,9 g du polymère pGluOBz- PheOC12 sont obtenus (soit 88% de rendement). La Mn (déterminé par GPC NMP) est de 12,5 kg/mol en équivalents PMMA.

### Etape 2 : Greffage sur l'amine terminale

10 g du précédent polymère sont ensuite dissous, à température ambiante, dans 361 ml de THF. Cette solution est refroidie à 0°C avant de procéder à l'ajout du chlorure d'acide laurique dilué dans du THF (0,66 g dans 7 ml de THF). Enfin, 0,5 ml de triéthylamine sont à leur tour ajoutés. Le milieu est ensuite remis à température ambiante pour une durée de réaction d'une heure. La formation rapide d'un précipité de chlorhydrate de triéthylamine est observée. A la fin de la réaction, le milieu réactionnel est versé dans 1,4 L d'éther diisopropylique. Le précipité est filtré, lavé à l'éthanol à 96% (2x360 ml) puis à l'éther diisopropylique (2x360 ml). Le produit est enfin séché dans une étuve sous vide à 40°C. 9,5 g du polymère t-pGluOBz-PheOC12/C12 sont obtenus.
La Mn (déterminé par GPC NMP) est de 11,9 kg/mol en équivalents PMMA.

### Etape 3 : Hydrolyse des esters benzyliques

8,9 g du précédent polymère sont dissous dans 68 ml de TFA à température ambiante. La solution est ensuite refroidie à 0°C avant de procéder à l'ajout au goutte à goutte du HBr (à 30% en poids dans l'acide acétique, 30 ml soit 4 équivalents). A la fin de l'ajout, le milieu est agité 4 heures à température ambiante. La fin de la réaction est vérifiée par RMN ¹H du milieu réactionnel dans le TFA-*d*. Le milieu réactionnel est ensuite versé dans un mélange eau-éther diisopropylique (50-50, volume total de 480 ml). Le polymère est ensuite filtré sur fritté et lavé à l'éthanol (2 x 100 ml) puis à l'éther diisopropylique (2 x 135 ml). Le polymère est séché 48 h à 40°C sous vide. 4,93 g du polymère t-pGluOH-PheOC12/C12 sont obtenus (soit 90% de rendement).
La présence des groupements hydrophobes greffés sur les extrémités du polymère est confirmée par RMN ¹H dans le TFA*-d* et le degré de polymérisation est de 36 (la valeur nominale est de 30). La Mn (déterminé par GPC NMP) est de 10,0 kg/mol en équivalents PMMA.

### Etape 4 : Mise en suspension du polymère dans l'eau

4,4 g du polymère précédent sont mis en suspension dans 150 ml d'eau déminéralisée. 31 ml d'une solution de NaOH 1N sont lentement ajoutés. La neutralisation est terminée lorsque tout le polymère s'est solubilisé et que le pH se situe autour de 7,4. On obtient une solution limpide à l'oeil et stable dans le temps à température ambiante.

### Exemple 3 : synthèse d'un t-pGluONa PheOC18/C18

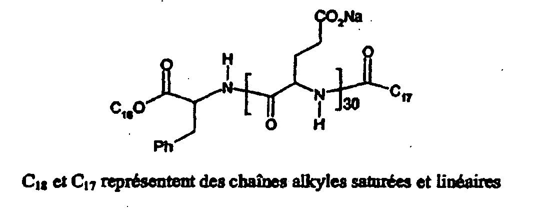

Ce polymère a été synthétisé selon le même procédé décrit dans l'exemple 2.
Le degré de polymérisation déterminé par RMN ¹H dans le TFA-*d* est de 38 (la valeur nominale est de 30). La Mn (déterminé par GPC NMP) est de 7,0 kg/mol en équivalents PMMA.

### Exemple 4 : synthèse d'un t-pGluONa PheOC18/T

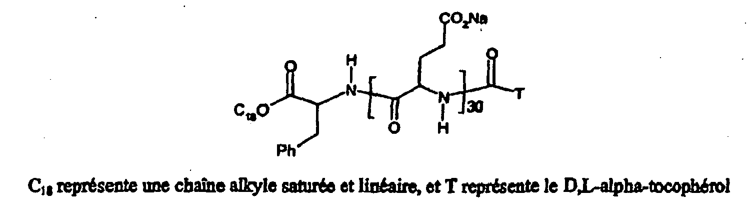

Ce polymère a été synthétisé selon le même procédé décrit dans l'exemple 2. Le greffage du groupement D,L-alpha-tocophérol est réalisé par réaction avec le dérivé chloroformiate correspondant. Le degré de polymérisation déterminé par RMN ¹H dans le TFA*-d* est de 36 (la valeur nominale est de 30). La Mn (déterminé par GPC NMP) est de 7,6 kg/mol en équivalents PMMA.

### Exemple 5 : Synthèse d'un composé comparatif C1, pGluONa C18

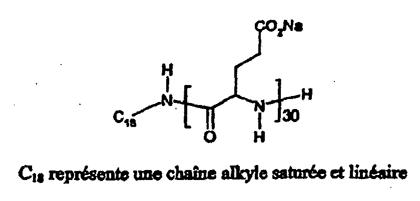

Ce polymère est obtenu par une synthèse équivalente à celle rapportée dans l'exemple 2. L'amorceur employé est dans ce cas la stéarylamine et aucune réaction de greffage n'est effectuée sur l'autre extrémité du polymère. En fin de synthèse, le polymère a les caractéristiques suivantes.

Le degré de polymérisation déterminé par RMN ¹R dans le TFA-*d* est de 32 (la valeur nominale est de 30). La Mn (déterminé par GPC NMP) est de 8,300 kg/mol en équivalents PMMA.

### Exemple 6 : Etude d'association avec l'insuline

On prépare une solution aqueuse contenant 10 mg de polymère par millilitre à pH 7,4 et 200 UI d'insuline (7,4 mg). On laisse incuber les solutions pendant deux heures à température ambiante et on sépare l'insuline libre de l'insuline associée par ultrafiltration (seuil à 100 kDa, 15 minutes sous 10000g à 18 °C). L'insuline libre récupérée dans le filtrat est ensuite dosée par CLHP (Chromatographie Liquide Haute Performance) et l'on déduit la quantité d'insuline associée. Les résultats sont donnés dans le tableau 1 ci-dessous.

**TABLEAU 1**

| Polymère | % association |
|---|---|
| Ex 3 | 93% |
| Ex 4 | 96% |
| Ex 5 (C1) | 37% |

Les résultats démontrent que les polymères de l'invention sont capables d'associer l'insuline pour donner des suspensions colloïdales de taille supérieure à 100 kDa et les taux d'association avec l'insuline sont très élevés. La capacité d'association de ces polymères les rend aptes à être utilisés comme agents de vectorisation.

## Revendications

1. Homopolyaminoacide amphiphile, linéaire et anionique **caractérisé en ce que** ses deux extrémités sont porteuses de groupement hydrophobes, identiques ou différents entre eux et **en ce qu'**il peut être symbolisé par la formule générale schématique suivante:
GH-X-PAA-Y-GH
avec:
- GH étant un Groupement Hydrophobe,
- X, Y indépendamment étant un lien correspondant à une liaison covalente ou à un radical polyvalent issu d'une entité chimique distincte du précurseur de GH,
- PAA étant une chaîne homopolyaminoacide hydrophile et anionique.

2. Homopolyaminoacide selon la revendication 1, **caractérisé en ce qu'**il comprend des unités d'alpha-L-glutamate et/ou d'alpha-L-glutamique ou des unités d'alpha-L-aspartate et/ou d'alpha-L-aspartique .

3. Homopolyaminoacide selon la revendication 1 ou 2, **caractérisé en ce que** les groupements hydrophobes sont choisis dans le groupe comprenant :
■ les alkyles linéaires ou ramifiés en C8 à C30 pouvant comporter éventuellement au moins une insaturation et/ou au moins un hétéroatome,
■ les alkylaryles ou arylalkyles en C8 à C30 pouvant comporter éventuellement au moins une insaturation et/ou au moins un hétéroatome,
■ et les (poly)cycliques en C8 à C30 pouvant comporter éventuellement au moins une insaturation et/ou au moins un hétéroatome.

4. Homopolyaminoacide selon l'une quelconque des revendications 1 à 3, **caractérisé en ce qu'**il répond à l'une des formules générales (I), (II) et (III) suivantes : 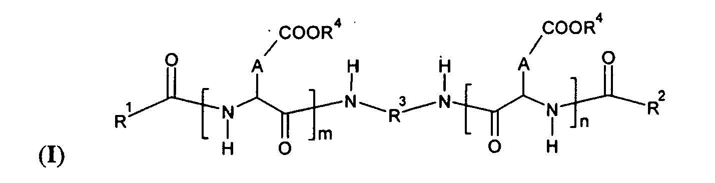 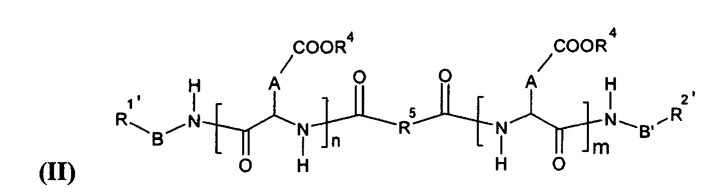 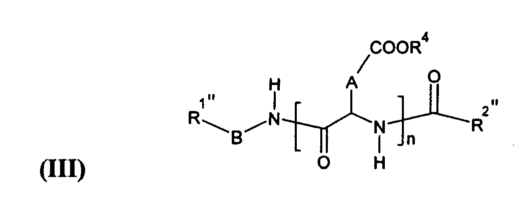 dans lesquelles :
■ R¹CO-, R²CO-, R^{1'}, R^{2'}, R^{1"} et R^{2"} représentent indépendamment un groupement hydrophobe en C8 à C30
■ R³ est un groupement alkyle linéaire en C2 à C6
■ R⁴ est un H ou une entité cationique, de préférence sélectionnée dans le groupe comprenant :
- les cations métalliques avantageusement choisis dans le sous-groupe comprenant : le sodium, le potassium, le calcium, le magnésium,
- les cations organiques avantageusement choisis dans le sous-groupe comprenant :
• les cations à base d'amine,
• les cations à base d'oligoamine,
• les cations à base de polyamine (la polyéthylèneimine étant particulièrement préférée),
• les cations à base d'acide(s) aminé(s) avantageusement choisis dans la classe comprenant les cations à base de lysine ou d'arginine,
- ou les polyaminoacides cationiques avantageusement choisis dans le sous-groupe comprenant la polylysine ou l'oligolysine,
■ R⁵ est un groupement alkyle, dialcoxy ou diamine en C2 à C6
■ A représente indépendamment un -CH₂- (unité aspartique) ou -CH₂-CH₂- (unité glutamique)
■ B est un lien formé par une liaison covalente directe ou un reste acide aminé répondant à la formule suivante : 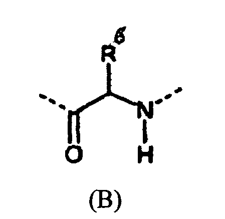
dans laquelle R⁶ est un radical caractéristique d'un acide aminé naturel, de préférence choisi dans le groupe comprenant : H (B étant alors un reste glycine), Méthyle (B étant alors un reste alanine), isobutyle (B étant alors un reste leucine), isopropyle (B étant alors un reste valine) ou CH₂Ph (B étant alors un reste phénylalanine)
■ n+m est défini comme le degré de polymérisation et varie de 3 à 1000, de préférence entre 20 et 300.

5. Homopolyaminoacide selon l'une quelconque des revendications 1 à 4, **caractérisé en ce que** le groupement hydrophobe GH est un groupement choisi dans le groupe comprenant les espèces suivantes : palmitate, stéarate, cholestéryle, tocophéryle.

6. Homopolyaminoacide selon l'une quelconque des revendications 1 à 5, **caractérisé en ce que** sa masse molaire se situe entre 2 000 et 100 000 g/mole, et de préférence entre 5 000 et 40 000 g/mole.

7. Composition pharmaceutique, cosmétique, diététique ou phytosanitaire comprenant au moins un homopolyaminoacide selon l'une quelconque des revendications 1 à 6.

8. Composition selon la revendication 7, **caractérisée en ce qu'**elle comprend au moins un principe actif.

9. Composition, notamment selon la revendication 8, **caractérisé en ce que** le principe actif est associé au(x) polyaminoacide(s) par une ou plusieurs liaisons autre(s) qu'une (ou que des) liaison(s) chimique(s) covalente(s).

10. Composition selon la revendication 8 ou 9, **caractérisée en ce que** le principe actif est une protéine, une glycoprotéine, une protéine liée à une ou plusieurs chaînes polyalkylèneglycol {de préférence PolyEthylèneGlycol (PEG) : "protéine-PEGylée"}, un polysaccharide, un liposaccharide, un oligonucléotide, un polynucléotide ou un peptide.

11. Composition selon la revendication 8 ou 9, **caractérisée en ce que** le principe actif est une petite molécule organique hydrophobe, hydrophile ou amphiphile.

12. Composition selon la revendication 7, 8 ou 9, **caractérisée en ce qu'**elle peut être administrée par voie orale, parentérale, nasale, vaginale, oculaire, sous-cutanée, intraveineuse, intramusculaire, intradermique, intra péritonéale, intracérébrale ou buccale.

13. Composition selon la revendication 7, 8 ou 9, **caractérisée en ce qu'**elle est sous forme d'un gel, d'une émulsion, de micelles, de nanoparticules, de microparticules, d'une poudre ou d'un film.

14. Composition selon la revendication 7, 8 ou 9, **caractérisée en ce qu'**elle est une suspension colloïdale de nanoparticules et/ou de microparticules et/ou de micelles de polyaminoacides, dans une phase aqueuse.

15. Composition selon la revendication 7, 8 ou 9, **caractérisée en ce qu'**elle est sous forme de solution dans un solvant biocompatible et **en ce qu'**elle peut être injectée par voie sous-cutanée, intramusculaire ou dans une tumeur.

16. Composition selon la revendication 7, 8 ou 9, **caractérisée en ce qu'**elle est apte à former un dépôt au site d'injection.

17. Procédé de préparation:
• de médicaments, en particulier pour administration orale, nasale, vaginale, oculaire, sous-cutanée, intraveineuse, intramusculaire, intradermique, intrapéritonéale ou intracérébrale, les principes actifs de ces médicaments pouvant être, notamment, des protéines, des glycoprotéines, des protéines liées à une ou plusieurs chaînes polyalkylèneglycol {par exemple PolyEthylèneGlycol (PEG), on parle alors de protéines "PEGylées"}, des peptides, des polysaccharides, des liposaccharides, des oligonucléotides, des polynucléotides et des petites molécules organiques hydrophobes, hydrophiles ou amphiphiles ;
• et/ou des nutriments ;
• et/ou de produits cosmétiques ou phytosanitaires ;
**caractérisé en ce qu'**il consiste essentiellement à mettre en oeuvre au moins un homopolyaminoacide selon la revendication 1 et/ou la composition selon la revendication 7, 8 ou 9.

18. Procédé de préparation d'un homopolyaminoacide tel que défini à l'une quelconque des revendications 4 à 6 de formule (I) **caractérisé en ce qu'**il est obtenu en faisant réagir les composés ci-dessous, Bz signifiant Benzyle, 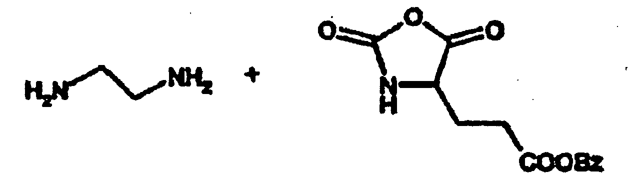 pour obtenir un composé de formule 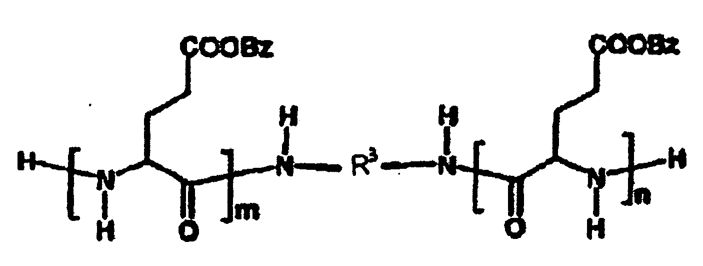 où R₃ = -(CH₃)₂ , n et m sont définis comme à la revendication 4 et le polymaminoacide est un polyglutamate, que l'on fait réagir avec un halogénure d'acyle de formule 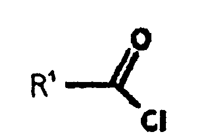 où R1 = C₁₇, pour obtenir un composé de formule 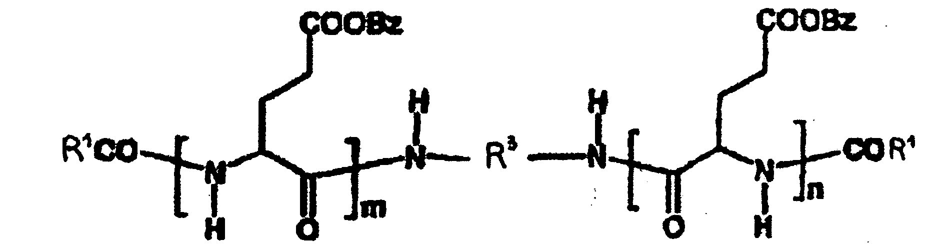 où R₁, R₃, Bz , n et m ont la signification déjà indiquée, permettant finalement d'obtenir un composé de formule (I) 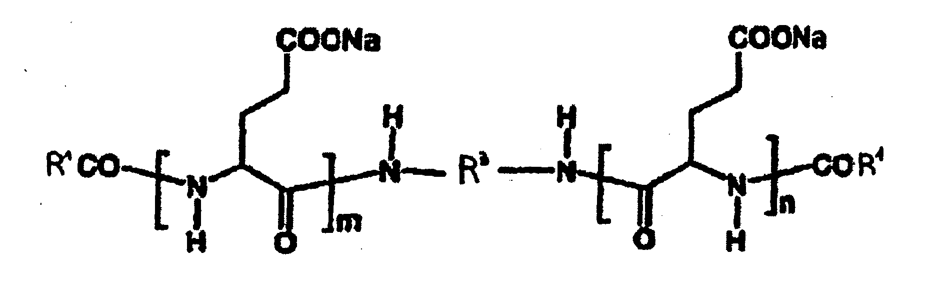 attendu.

19. Procédé de préparation d'un homopolyaminoacide de formule (II) tel que défini à l'une quelconque des revendications 4 à 6 dans lequel R1' est le dodécanol et B est la leucine, **caractérisé en ce qu'**il est obtenu par la séquence réactionnelle suivante : initiation d'une polymérisation de NCA-Glu-O-Bz par un groupement hydrophobe ayant une fonction amine, NCA signifiant anhydride de N-carboxy-aminoacide et Bz signifiant Benzyle, pour obtenir un produit intermédiaire de type (II-A), terminé par une fonction aminé, 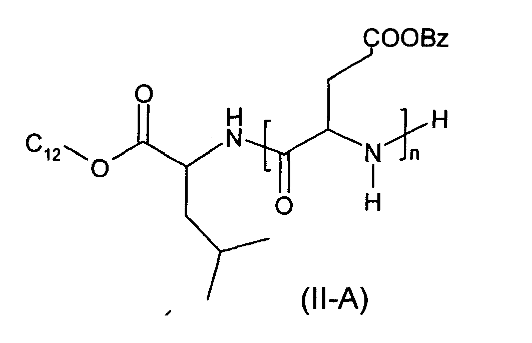 puis couplage dudit produit intermédiaire (II-A) avec un composé difonctionnel approprié tel qu'un dichlorure d'acide, un diisocyanate ou un dichloroformiate pour obtenir un composé de formule (II) attendu.

20. Procédé de préparation d'un homopolyaminoacide de formule (III) tel que défini à l'une quelconque des revendications 4 à 6 dans lequel R1' est le dodécanol et B est la leucine, **caractérisé en ce qu'**il est obtenu par réaction d'un intermédiaire (II-A) tel que défini à la revendication 19 avec un groupement hydrophobe fonctionnalisé tel qu'un chlorure d'acide ou un chloroformiate, pour obtenir un composé de formule (III) attendu.

## Claims

1. Anionic, linear, amphiphilic homopolyamino acid, **characterized in that** its two ends carry hydrophobic groups that are identical to or different from one another, and **in that** it can be symbolized by the following schematic general formula:
HG-X-PAA-Y-HG
in which:
- HG is a hydrophobic group,
- X and Y independently are a link corresponding to a covalent bond or to a polyvalent radical derived from a chemical entity different from the precursor of HG, and
- PAA is an anionic, hydrophilic homopolyamino acid chain.

2. Homopolyamino acid according to claim 1, **characterized in that** it comprises alpha-L-glutamate and/or alpha-L-glutamic units or alpha-L-aspartate and/or alpha-L-aspartic units.

3. Homopolyamino acid according to claim 1 or 2, **characterized in that** the hydrophobic groups are selected from the group comprising:
■ linear or branched C8 to C30 alkyls which can optionally contain at least one unit of unsaturation and/or at least one heteroatom,
■ C8 to C30 alkylaryls or arylalkyls which can optionally contain at least one unit of unsaturation and/or at least one heteroatom,
■ and C8 to C30 (poly)cyclic groups which can optionally contain at least one unit of unsaturation and/or at least one heteroatom.

4. Homopolyamino acid according to any one of claims 1 to 3, **characterized in that** it has one of the general formulae (I), (II) and (III) below: 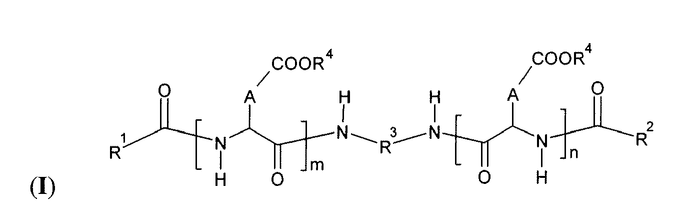 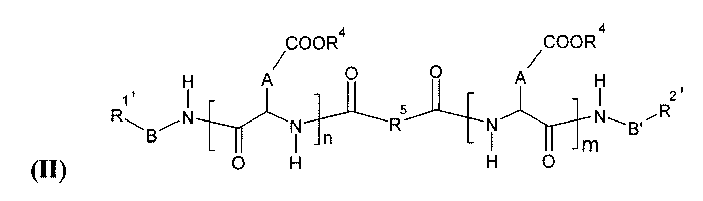 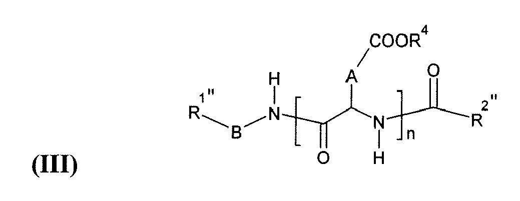 in which:
■ R¹CO-, R²CO-, R^{1'}, R^{2'}, R^{1"} and R^{2"} independently are a C8 to C30 hydrophobic group;
■ R³ is a linear C2 to C6 alkyl group;
■ R⁴ is H or a cationic entity preferably selected from the group comprising:
- metal cations advantageously selected from the subgroup comprising sodium, potassium, calcium and magnesium,
- organic cations advantageously selected from the subgroup comprising:
• cations based on amine,
• cations based on oligoamine,
• cations based on polyamine (polyethylenimine being particularly preferred), and
• cations based on amino acid(s) and advantageously selected from the class comprising cations based on lysine or arginine; and
- cationic polyamino acids advantageously selected from the subgroup comprising polylysine and oligolysine;
■ R⁵ is a C2 to C6 alkyl, dialkoxy or diamine group;
■ A independently is -CH₂- (aspartic unit) or -CH₂-CH₂- (glutamic unit);
■ B is a link formed of a direct covalent bond or an amino acid residue of the formula below: 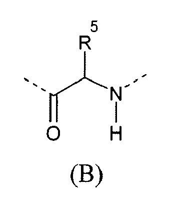
in which R⁵ is a radical characteristic of a natural amino acid and preferably selected from the group comprising H (in which case B is a glycine residue), methyl (in which case B is an alanine residue), isobutyl (in which case B is a leucine residue), isopropyl (in which case B is a valine residue) and CH₂Ph (in which case B is a phenylalanine residue); and
■ n + m is defined as the degree of polymerization and varies from 3 to 1000, preferably between 20 and 300.

5. Homopolyamino acid according to any one of claims 1 to 4, **characterized in that** the hydrophobic group HG is a group selected from the group comprising the following species: palmitate, stearate, cholesteryl and tocopheryl.

6. Homopolyamino acid according to any one of claims 1 to 5, **characterized in that** its molecular weight is between 2000 and 100,000 g/mol, preferably between 5000 and 40,000 g/mol.

7. Pharmaceutical, cosmetic, dietetic or phytosanitary composition comprising at least one homopolyamino acid according to any one of claims 1 to 6.

8. Composition according to claim 7, **characterized in that** it comprises at least one active principle.

9. Composition, especially according to claim 8, **characterized in that** the active principle is associated with the polyamino acid(s) by one or more bonds other than covalent chemical bonds.

10. Composition according to claim 8 or 9, **characterized in that** the active principle is a protein, a glycoprotein, a protein bonded to one or more polyalkylene glycol chains {preferably polyethylene glycol (PEG) chains: "PEGylated protein"}, a polysaccharide, a liposaccharide, an oligonucleotide, a polynucleotide or a peptide.

11. Composition according to claim 8 or 9, **characterized in that** the active principle is a small hydrophobic, hydrophilic or amphiphilic organic molecule.

12. Composition according to claim 7, 8 or 9, **characterized in that** it can be administered by the oral, parenteral, nasal, vaginal, ocular, subcutaneous, intravenous, intramuscular, intradermal, intraperitoneal, intracerebral or buccal route.

13. Composition according to claim 7, 8 or 9, **characterized in that** it is in the form of a gel, an emulsion, micelles, nanoparticles, microparticles, a powder or a film.

14. Composition according to claim 7, 8 or 9, **characterized in that** it is a colloidal suspension of nanoparticles and/or microparticles and/or micelles of polyamino acids in an aqueous phase.

15. Composition according to claim 7, 8 or 9, **characterized in that** it is in the form of a solution in a biocompatible solvent and **in that** it can be injected by the subcutaneous or intramuscular route or into a tumor.

16. Composition according to claim 7, 8 or 9, **characterized in that** it is capable of forming a depot at the injection site.

17. Process for the preparation of:
• drugs, particularly for administration by the oral, nasal, vaginal, ocular, subcutaneous, intravenous, intramuscular, intradermal, intraperitoneal or intracerebral route, it being possible in particular for the active principles of these drugs to be proteins, glycoproteins, proteins bonded to one or more polyalkylene glycol chains {e.g. polyethylene glycol (PEG) chains, in which case the term "PEGylated" proteins is used}, peptides, polysaccharides, liposaccharides, oligonucleotides, polynucleotides and small hydrophobic, hydrophilic or amphiphilic organic molecules;
• and/or nutriments;
• and/or cosmetic or phytosanitary products,
**characterized in that** it consists essentially in using at least one homopolyamino acid according to claim 1 and/or the composition according to claim 7, 8 or 9.

18. A process for the preparation of a homopolyaminoacid defined in any of claims 4 to 6 of formula (I) wherein said homopolyaminoacid is obtained by reacting the compounds hereunder where Bz = Benzyl 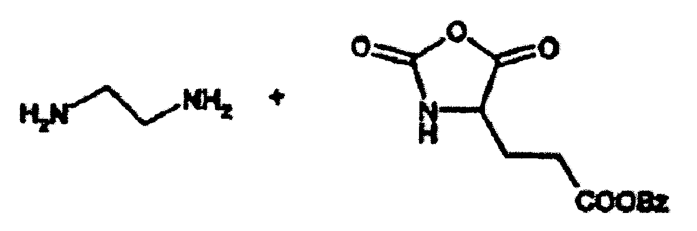 to obtain a compound of formula 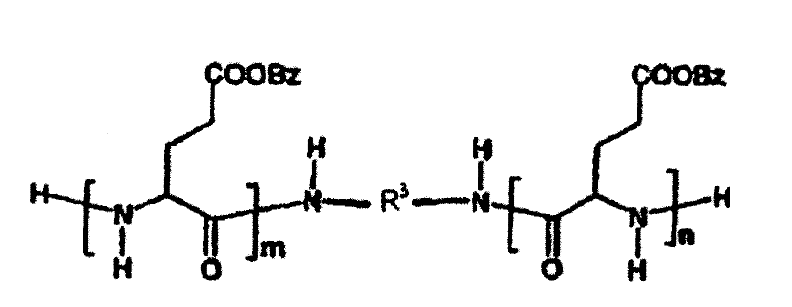 wherein R₃= -(CH₃)₂, ,n and m are defined as in claim 4 and the polyaminoacid is a glutamate, which is reacted with a acyl halide of formula 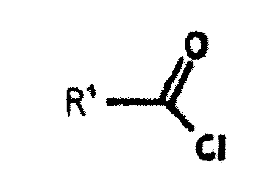 where R1 = C₁₇, to obtain a compound of formula 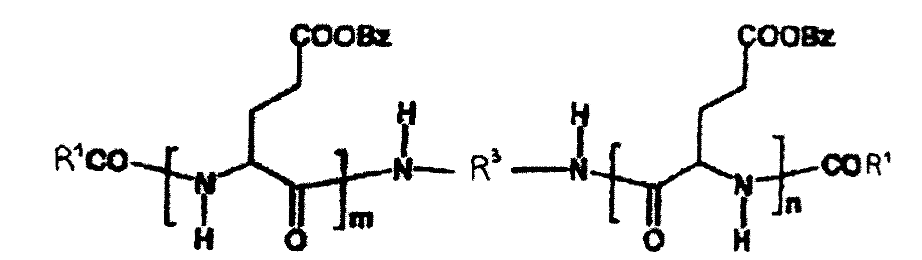 where R¹ , R³ , Bz, n and m have the meaning given above, to finally obtain a desired compound of formula (I)

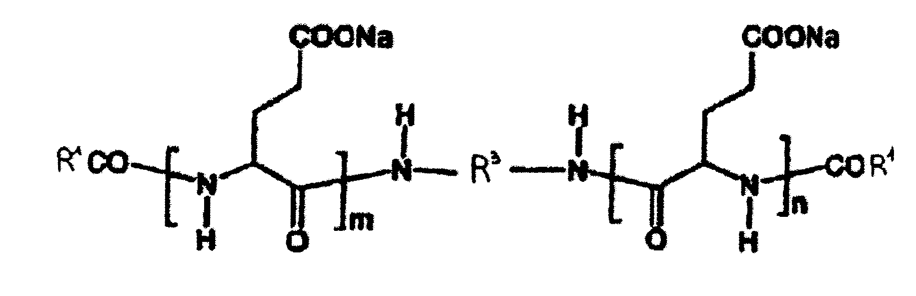

19. A process for the preparation of a homopolyaminoacid defined in any of claims 4 to 6 of formula (II) wherein R1' is dodecanol and B is leucine, and wherein said homopolyaminoacid is obtained according to the following scheme: initiation of a polymerization of NCA-Glu-O-Bz by a hydrophobic group carrying an amine group , wherein NCA means N-carboxy aminoacid anhydride and Bz = Benzyl to give an intermediate (II-A) having a terminal amine group, 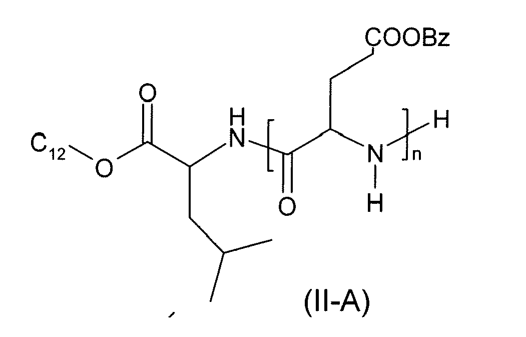 followed by coupling of said intermediate (II-A) with an appropriate difunctional compound such as an acid dichloride, a diisocyanate or a dichloroformiate to obtain a desired compound of formula (II).

20. A process for the preparation of a homopolyaminoacid defined in any of claims 4 to 6 of formula (II) wherein R1' is dodecanol and B is leucine, and wherein said homopolyaminoacid is obtained by reacting an intermediate (II-A) defined in claim 19 with a functionalized hydrophobic group such as an acid chloride or a chloroformate to obtain a desired compound of formula (III).

## Patentansprüche

1. Anionische, lineare und amphiphile Homopolyaminosäure, **dadurch gekennzeichnet, dass** ihre beiden Enden Träger hydrophober Gruppen sind, welche gleich oder verschieden voneinander sind, und die durch folgende allgemeine schematische Formel ausgedrückt werden kann:
GH-X-PAA-Y-GH
wobei:
- GH eine hydrophobe Gruppe ist,
- X, Y unabhängig voneinander eine Verknüpfung sind, die einer kovalenten Bindung oder einem mehrwertigen Radikal, das aus einer chemischen Einheit stammt, die verschieden von der Vorstufe von GH ist, entspricht,
- PAA eine anionische und hydrophile Homopolyaminosäurekette ist.

2. Homopolyaminosäure gemäß Anspruch 1, **dadurch gekennzeichnet, dass** sie alpha-L-Glutamat- und/oder alpha-L-Glutamin-Einheiten oder alpha-L-Aspartat- und/oder alpha-L-Asparagin-Einheiten umfasst.

3. Homopolyaminosäure gemäß Anspruch 1 oder 2, **dadurch gekennzeichnet, dass** die hydrophoben Gruppen ausgewählt sind aus der Gruppe umfassend:
- lineare oder verzweigte C8 bis C30-Alkyle, die gegebenenfalls mindestens eine ungesättigte Bindung und/oder mindestens ein Heteroatom aufweisen können,
- C8 bis C30-Alkylaryle oder -Arylalkyle, die gegebenenfalls mindestens eine ungesättigte Bindung und/oder mindestens ein Heteroatom aufweisen können, und
- C8 bis C30 (Poly)Cyclen, die gegebenenfalls mindestens eine ungesättigte Bindung und/oder mindestens ein Heteroatom aufweisen können.

4. Homopolyaminosäure gemäß einem der Ansprüche 1 bis 3, **dadurch gekennzeichnet, dass** sie einer der folgenden allgemeinen Formeln (I), (II) und (III) entspricht: 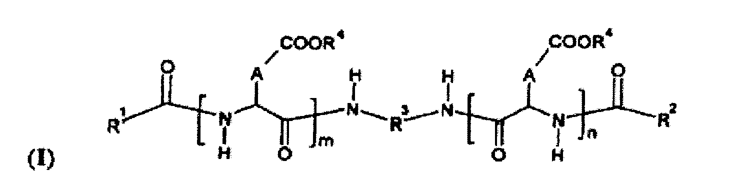 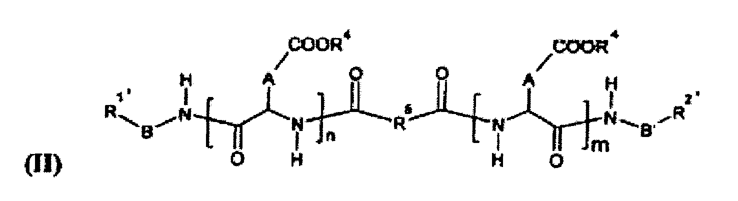 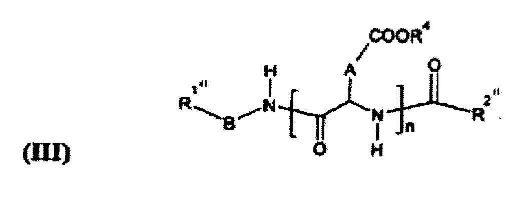 in denen:
- R¹CO-, R²CO-, R^{1'}, R^{2'}, R^{1"} und R^{2"} unabhängig voneinander eine hydrophobe C8 bis C30-Gruppe darstellen,
- R³ eine lineare C2 bis C6-Alkylgruppe ist,
- R⁴ ein H oder eine kationische Einheit ist, die vorzugsweise ausgewählt ist aus der Gruppe umfassend:
- metallische Kationen, die vorteilhaft ausgewählt sind aus der Untergruppe, die umfasst: Natrium, Kalium, Calcium, Magnesium,
- organische Kationen, die vorteilhaft ausgewählt sind aus der Untergruppe, die umfasst:
• Kationen auf Basis von Amin,
• Kationen auf Basis von Oligoamin,
• Kationen auf Basis von Polyamin (wobei Polyethylenimin besonders bevorzugt ist),
• Kationen auf Basis von Aminosäure(n), die vorteilhaft ausgewählt sind aus der Klasse, die Kationen auf Basis von Lysin oder Arginin umfasst,
- oder kationische Polyaminosäuren, die vorteilhaft ausgewählt sind aus der Untergruppe, die Polylysin oder Oligolysin umfasst,
- R⁵ eine C2 bis C6-Alkyl-, -Dialkoxy-, oder -Diamin-Gruppe ist,
- A unabhängig eine -CH₂- (Asparagineinheit) oder -CH₂-CH₂- (Glutamineinheit) darstellt,
- B eine Verknüpfung ist, die durch eine direkte kovalente Bindung oder einen Aminosäurerest, der folgender Formel entspricht, gebildet wird, 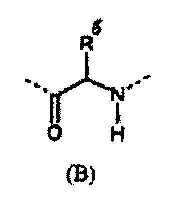 in der R6 ein Radikal ist, das von einer natürlichen Aminosäure **gekennzeichnet** wird, vorzugsweise ausgewählt aus der Gruppe umfassend: H (B wäre dann ein Glycinrest), Methyl (B wäre dann ein Alaninrest), Isobutyl (B wäre dann ein Leucinrest), Isopropyl (B wäre dann ein Valinrest) oder CH₂Ph (B wäre dann ein Phenylalaninrest),
- n+m als Polymerisationsgrad definiert ist und zwischen 3 und 1000, vorzugsweise zwischen 20 und 300 variiert.

5. Homopolyaminosäure gemäß einem der Ansprüche 1 bis 4, **dadurch gekennzeichnet, dass** die hydrophobe Gruppe GH eine Gruppe ist, die ausgewählt ist aus der Gruppe, die folgende Spezies umfasst: Palmitat, Stearat, Cholesteryl, Tocopheryl.

6. Homopolyaminosäure gemäß einem der Ansprüche 1 bis 5, **dadurch gekennzeichnet, dass** ihre molare Masse zwischen 2.000 und 100.000 g/mol und vorzugsweise zwischen 5.000 und 40.000 g/mol liegt.

7. Pharmazeutische, kosmetische, diätetische oder Pfilanzenschutz-Zusammensetzung, die mindestens eine Homopolyaminosäure gemäß einem der Ansprüche 1 bis 6 umfasst.

8. Zusammensetzung gemäß Anspruch 7, **dadurch gekennzeichnet, dass** sie mindestens einen Wirkstoff umfasst.

9. Zusammensetzung, insbesondere gemäß Anspruch 8, **dadurch gekennzeichnet, dass** der Wirkstoff mit Polyaminosäure(n) über eine oder mehrere Bindungen assoziiert ist, die verschieden sind von einer oder mehreren kovalenten chemischen Bindungen.

10. Zusammensetzung gemäß Anspruch 8 oder 9, **dadurch gekennzeichnet, dass** der Wirkstoff ein Protein, ein Glykoprotein, ein Protein, das mit einer oder mehreren Polyalkylenglykolketten verknüpft ist {vorzugsweise Polyethylenglykol (PEG):
"PEGyliertes Protein"}, ein Polysaccharid, ein Liposaccharid, ein Oligonukleotid, ein Polynukleotid oder ein Peptid ist.

11. Zusammensetzung gemäß Anspruch 8 oder 9, **dadurch gekennzeichnet, dass** der Wirkstoff ein hydrophobes, hydrophiles oder amphiphiles kleines organisches Molekül ist.

12. Zusammensetzung gemäß Anspruch 7, 8 oder 9, **dadurch gekennzeichnet, dass** sie auf oralem, parenteralem, nasalem, vaginalem, okularem, subkutanem, intravenösem, intramuskulärem, intradermischem, intraperitonealem, intracerebralem Wege oder über den Mund verabreicht werden kann.

13. Zusammensetzung gemäß Anspruch 7, 8 oder 9, **dadurch gekennzeichnet, dass** sie in Form eines Gels, einer Emulsion, von Mizellen, von Nanopartikein, von Mikropartikeln, eines Pulvers oder eines Films vorliegt.

14. Zusammensetzung gemäß Anspruch 7, 8 oder 9, **dadurch gekennzeichnet, dass** sie eine kolloidale Suspension von Nanopartikeln und/oder Mikropartikeln und/oder Mizellen von Polyaminosäuren in einer wässrigen Phase ist.

15. Zusammensetzung gemäß Anspruch 7, 8 oder 9, **dadurch gekennzeichnet, dass** sie in Form einer Lösung in einem biokompatiblen Lösungsmittel vorliegt und dass sie auf subkutanem, intramuskulärem Wege oder in einen Tumor injiziert werden kann.

16. Zusammensetzung gemäß Anspruch 7, 8 oder 9, **dadurch gekennzeichnet, dass** sie in der Lage ist, am Ort der Injektion ein Depot zu bilden.

17. Verfahren zur Herstellung von:
- Medikamenten, insbesondere zur oralen, nasalen, vaginalen, okularen, subkutanen, intravenösen, intramuskulären, intradermischen, intraperitonealen oder intracerebralen Verabreichung, wobei die Wirkstoffe dieser Medikamente insbesondere Proteine, Glykoproteine, Proteine, die mit einer oder mehreren Polyalkylenglykolketten verknüpft sind {zum Beispiel Polyethylenglykol (PEG), man spricht dann von "PEGylierten" Proteinen}, Peptide, Polysaccharide, Liposaccharide, Oligonukleotide, Polynukleotide und hydrophobe, hydrophile oder amphiphile kleine organische Moleküle sein können; und/oder
- Nährstoffen; und/oder
- kosmetischen oder Pflanzenschutz-Produkten;
**dadurch gekennzeichnet, dass** es im Wesentlichen darin besteht, mindestens eine Homopolyaminosäure gemäß Anspruch 1 und/oder die Zusammensetzung gemäß Anspruch 7, 8 oder 9 einzusetzen.

18. Verfahren zur Herstellung einer Homopolyaminosäure der Formel (I), wie sie in einem der Ansprüche 4 bis 6 definiert ist, **dadurch gekennzeichnet, dass** sie erhalten wird, indem die nachstehenden Verbindungen zur Reaktion gebracht werden, wobei Bz Benzyl bedeutet, 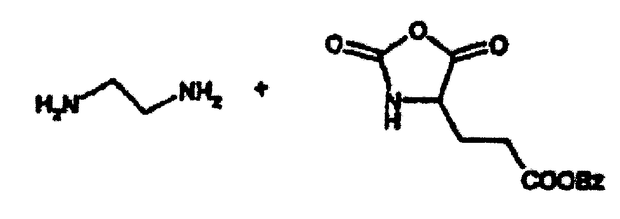 um eine Verbindung der Formel 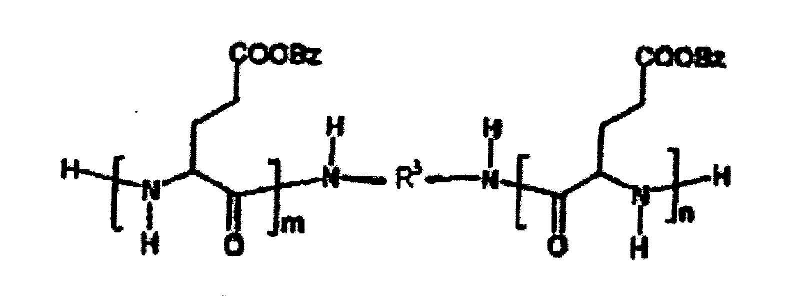 zu erhalten, wobei R³= -(CH₃)₂, n und m wie in Anspruch 4 definiert sind und die Polyaminosäure ein Polyglutamat ist, das mit einem Acylhalogenid der Formel 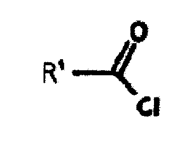 zur Reaktion gebracht wird, wobei R1 = C₁₇, , um eine Verbindung der Formel 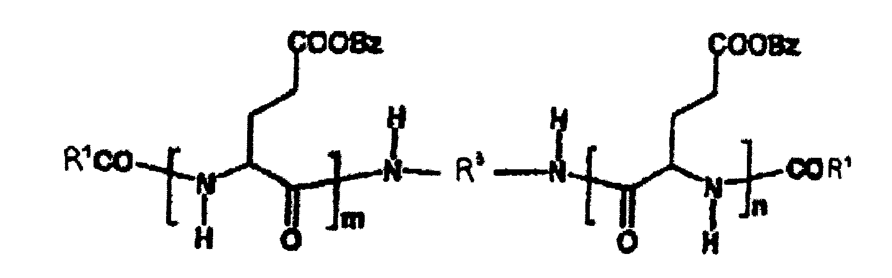 zu erhalten, wobei R1, R3, Bz, n und m die bereits angegebene Bedeutung haben;, um schließlich eine Verbindung der Formel (I): 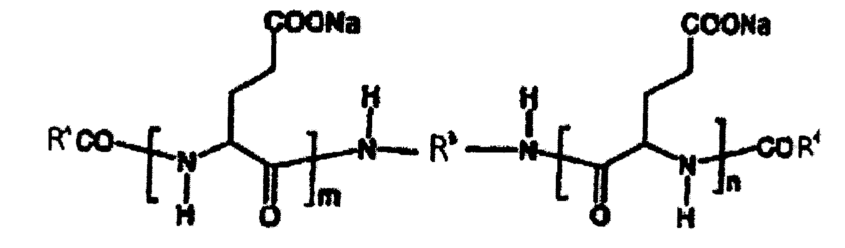 zu erhalten.

19. Verfahren zur Herstellung einer Homopolyaminosäure der Formel (II), wie sie in einem der Ansprüche 4 bis 6 definiert ist, in der R1 Dodecanol und B Leucin bedeutet, **dadurch gekennzeichnet, dass** sie aus der folgenden Reaktionsfolge erhalten wird: Initiation einer Polymerisation von
NCA-Glu-O-Bz durch eine hydrophobe Gruppe, die ein Aminrest besitzt, wobei NCA ein Anhydrid von N-Carboxyaminosäure bedeutet und Bz Benzyl bedeutet, um ein Zwischenprodukt vom Typ (II-A) zu erhalten, das endständig einen Aminrest aufweist, 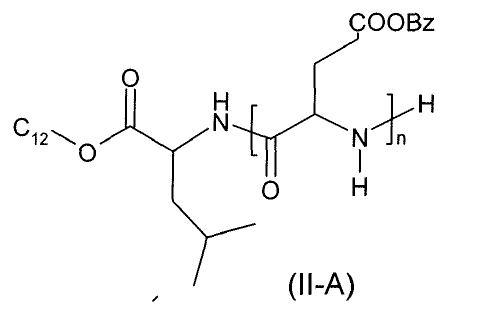 anschließend einer Kopplung dieses Zwischenprodukts (II-A) mit einer passenden difunktionellen Verbindung, wie etwa einem Säuredichlorid, einem Düsocyanat oder einem Dichlorformiat, um eine erwartete Verbindung der Formel (II) zu erhalten.

20. Verfahren zur Herstellung einer Homopolyaminosäure der Formel (III), wie sie in einem der Ansprüche 4 bis 6 definiert ist, in der R1 Dodécanol und B Leucin bedeutet, **dadurch gekennzeichnet, dass** sie durch Reaktion eines Zwischenprodukts (II-A), wie es in Anspruch 19 definiert ist, mit einer funktionalisierten hydrophoben Gruppe, wie etwa einem Säurechlorid oder einem Chlorformiat erhalten wird, um eine Verbindung der Formel (III) zu erhalten.
